# FASCICULE DE BREVET EUROPEEN

(11) **EP 1 865 874 B1**
(45) Date de publication et mention de la délivrance du brevet: **16.12.2009**
(21) Numéro de dépôt: 06743584.2
(22) Date de dépôt: 16.03.2006
(51) Int. Cl.: A61B 19/08

(54) **DISPOSITIF POUR L' ARTHROSCOPIE**
VORRICHTUNG FÜR DIE ARTHROSKOPIE
DEVICE FOR ARTHROSCOPY

(30) Priorité: 18.03.2005 FR 0502692
(43) Date de publication de la demande: 19.12.2007
(73) Titulaire: VYGON, F-95440 Ecouen (FR)
(72) Inventeur: LESTOQUOY, Patrick, F-59551 Attiches (FR); ALGRAIN, Isabelle, F-95380 Puiseux en France (FR)
(74) Mandataire: Le Forestier, Eric
(86) Numéro de dépôt international: PCT/FR2006/000586
(87) Numéro de publication internationale: WO 2006/097635

(56) Documents cités:
- EP-A- 0 249 832
- WO-A-98/22037
- US-A- 3 565 067
- US-A- 3 750 664
- US-A- 3 766 913
- US-A- 3 930 497
- US-A- 5 074 316
- US-A- 5 349 965
- US-A1- 2003 121 522
- US-B1- 6 199 553
- US-B1- 6 824 853

## Description

L'invention concerne l'arthroscopie d'une articulation, par exemple pour l'arthroscopie du genou.

Pour réaliser cette arthroscopie, on utilise communément un champ, généralement grand et rectangulaire qui présente une fenêtre découpée dans une zone élastique située vers le centre du champ.

Le chirurgien plie la jambe du patient (couché sur le dos) pour amener l'articulation par exemple le genou, à la verticale. Il engage la fenêtre du champ sur le genou. Le genou rentre dans la fenêtre grâce à l'élasticité de celle-ci, et les bords de la fenêtre assurent l'étanchéité autour du genou.

Le reste du champ est déployé sur le patient et la table.

Lors de l'arthroscopie, en général, deux canules sont introduites dans le genou, l'une d'elles sert en particulier à injecter de l'eau saline régulièrement dans l'articulation, l'autre à visualiser et à opérer (enlèvement de fragments de cartilage).

Durant une opération, environ trois litres d'eau saline sont injectés dans le genou, ils ressortent bien entendu et s'écoulent sur le champ jusqu'à retomber au sol.

Le chirurgien a les pieds dans l'eau et il faut nettoyer entre chaque opération.

Des solutions ont été imaginées pour capter l'eau au niveau du genou, comme par exemple la création d'une poche de recueil (brevet US 4 974 604), mais ce n'est pas pratique. En effet, durant l'arthroscopie, le chirurgien bouge fortement le genou et la jambe pour faciliter le travail des outils introduits en fonction des images caméra qu'il doit regarder en permanence.

La poche est gênante et peut se vider d'un seul coup en conséquence d'un appui accidentel de l'opérateur.

La présente invention vise à recueillir l'eau en évitant les inconvénients précités.

Un dispositif pour recueillir l'eau qui est injectée dans une articulation au cours d'une arthroscopie est constitué d'un champ muni de la fenêtre, ou " champ principal ", d'une surface suffisante pour recouvrir l'articulation et descendre sensiblement jusqu'au niveau du sol lorsque le patient est couché sur le dos sur une table, et qui présente une zone (3) en matériau élastique qui détermine une fenêtre (4) pour le passage de l'articulation au travers de la fenêtre avec étanchéité latérale, et d'un champ supplémentaire (2) très hydrophile qui prolonge vers le bas le champ principal pour récupérer au sol l'eau saline qui a été injectée dans l'articulation, et qui est séparé du champ principal par une barrière (5) pour les germes.

On connaît des champs chirurgicaux à fenêtre comportant des zones absorbantes (par exemple brevet US 3 766 913 55) mais ces champs ne sont pas adaptés à l'arthroscopie.

Le document US 5 349 965 décrit un système d'évacuation de fluide lors d'une opération chirurgicale.

Le document US 6 824 853 décrit un bac pour recueillir du liquide destiné en particulier à l'application en chirurgie et comportant un fond en matériau absorbant.

On décrira ci-après un dispositif conforme à l'invention, et destiné à l'arthroscopie du genou, en référence aux figures du dessin joint sur lequel :
- la figure 1 est une vue en plan d'un champ selon un premier mode de réalisation ;
- la figure 2 est une vue latérale schématique du champ de la figure 1, en service ;
- la figure 3 est une vue en plan d'une variante de réalisation du champ ;
- la figure 4 est une vue en coupe d'une variante de réalisation d'un champ supplémentaire.

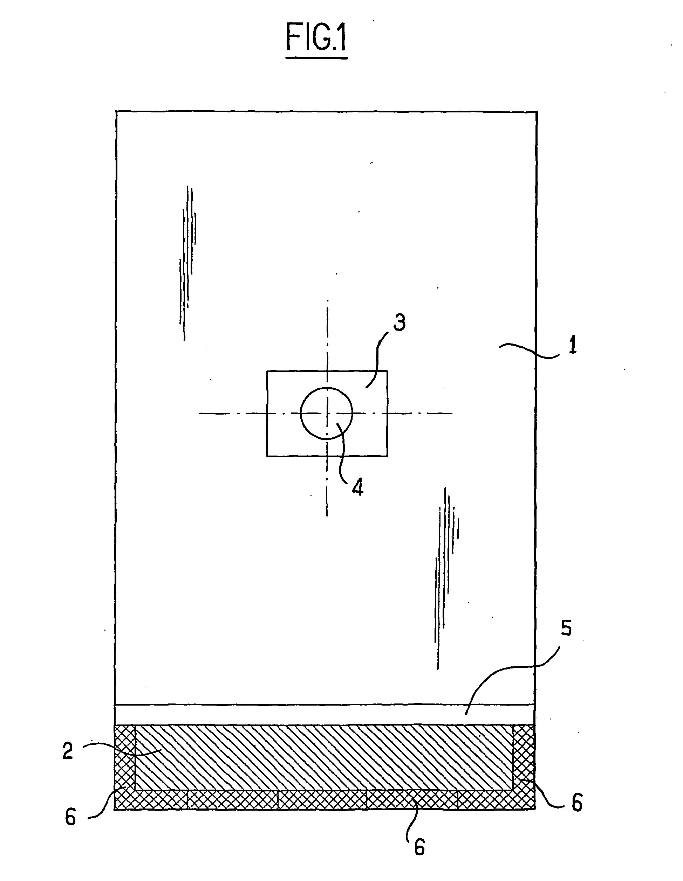

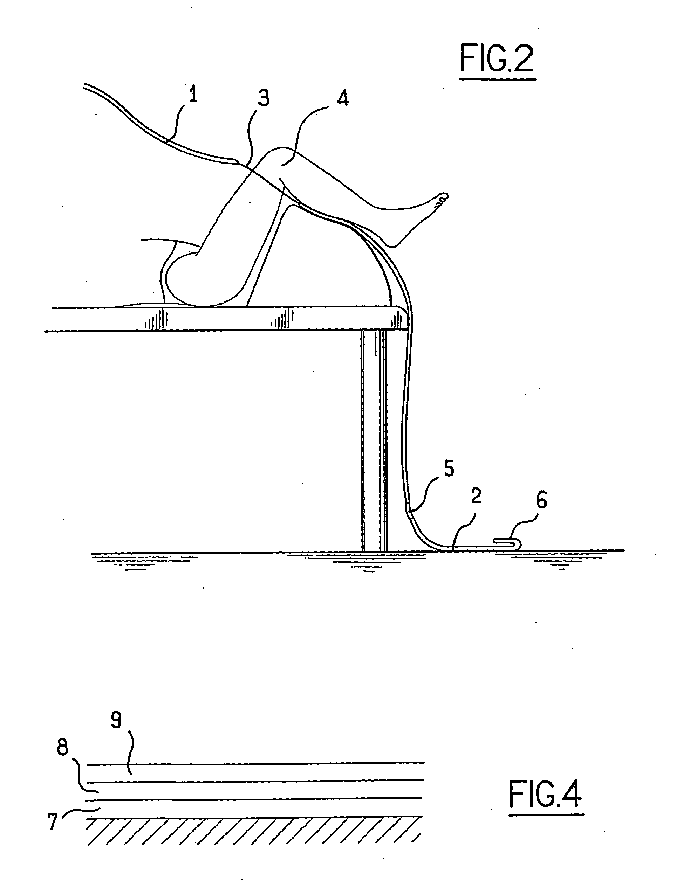

Le champ représenté sur les figures 1 et 2 comprend un champ principal (1) et un champ supplémentaire (2).

Le champ principal (1), en soi connu, est d'une surface suffisante pour recouvrir le genou du patient et descendre sensiblement jusqu'au niveau du sol, lorsque le patient est couché sur le dos sur une table, jambe pliée en sorte que soit amené sensiblement à la verticale. Ce champ comporte en son centre une zone rectangulaire (3) en matériau élastique qui détermine une fenêtre circulaire (4) pour le passage du genou au travers de la fenêtre avec étanchéité latérale.

La surface du champ principal est dans cet exemple d'environ 2 x 3 mètres.

La zone rectangulaire (3) est constituée par exemple d'une feuille d'un matériau très élastique ; le reste du champ est constitué par exemple de façon en soi connue, d'un complexe de deux couches comprenant un film imperméable (polyéthylène par exemple) et d'une couche de non-tissé constitué par exemple de fibres de polypropylène.

Ce champ principal est légèrement hydrophile, capable d'absorber environ 0,25 litre d'eau/m².

Dans une variante, le champ principal est constitué par un non-tissé hydrophobe avec une zone de renfort au niveau site opératoire comportant un non-tissé absorbant (base Spunlace par exemple) et un film polyéthylène, cette zone ayant une capacité absorption d'eau d'environ 0,45l/m².

Le non-tissé hydrophobe est par exemple de base SPUNLACE ou SMS.

Le champ supplémentaire (2) prolonge vers le bas le champ principal pour reposer au sol.

Ce champ (2) est isolé du premier par une bande (5) en matière plastique (polyéthylène) qui fait office de barrière pour les germes, et il est composé, par exemple, d'un film imperméable, par exemple en polyéthylène, sur laquelle sont rapportées et fixées, par exemple par collage, une couche très absorbante et une couche en non-tissé, composée par exemple de fibres de polypropylène. La couche très absorbante en fibres enchevêtrées est constituée par exemple d'un réseau de fibres de cellulose et de fibres synthétiques enchevêtrées, de longueur d'environ 15mm, associées à un milieu en poudre superabsorbant.

Il existe une grande variété de milieux en poudre surperabsorbants, par exemple des polyacrylates, des sels d'ammonium quaternaire, et autres.

Le volume de la couche très absorbante est le plus souvent calculé pour permettre de stocker au moins un litre d'eau par m² et, de préférence jusqu'à trois ou quatre litres d'eau au m². Au cas où l'eau arriverait par jets en quantités importantes, des rabats (6), formant gouttières, peuvent être prévus sur trois côtés du champ, en sorte que l'eau ne puisse s'écouler hors du champ supplémentaire. Pour éviter que le chirurgien ne se prenne les pieds dans ce rabat (qui peut bailler par endroit), un point de soudure ou de collage est réalisé à intervalles réguliers pour fixer les rabats au champ.

Le champ supplémentaire (2) a une largeur d'environ 0,4 mètre et la bande (5) a une largeur d'environ 0,10 mètres. La bande et le champ s'étendent au moins sur toute la longueur du côté du champ principal qu'ils prolongent.

L'eau qui s'écoule autour du genou est captée par le champ supplémentaire au sol, le chirurgien n'a plus les pieds dans l'eau, il n'est pas gêné pour ses manipulations, et le bloc est facilement nettoyable entre chaque opération. Le surcoût du champ reste modique.

L'Airlaid est un non-tissé composé de fibres lognues en cellulose, enchevêtrées les unes aux autres, très absorbant (et utilisé déjà en angiographie mais avec un grammage/m² plus faible, 80 à 85 gr/m²), légèrement plus rigide.

Le non-tissé voie sèche est composé de fibres courtes en polyester de cellulose liées chimiquement. Il est moins absorbant que l'Airlaid, et évite l'impression de marcher sur une éponge.

On comprendra dans la pratique, lorsque le champ principal et le champ supplémentaire comporte une même base imperméable, ces deux champs, ainsi que la barrière pour les germes peut être constitués par une même feuille en polyéthylène qui présente :
- une zone supérieure agencée pour constituer le champ principal ;
- une zone intermédiaire qui constitue la barrière anti-germe ;
- une zone inférieure agencée pour constituer le champ supplémentaire.

L'invention n'est pas limitée aux réalisations qui ont été décrites.

Le champ de l'invention est adaptable à l'arthroscopie d'articulations autres que le genou, l'épaule par exemple.

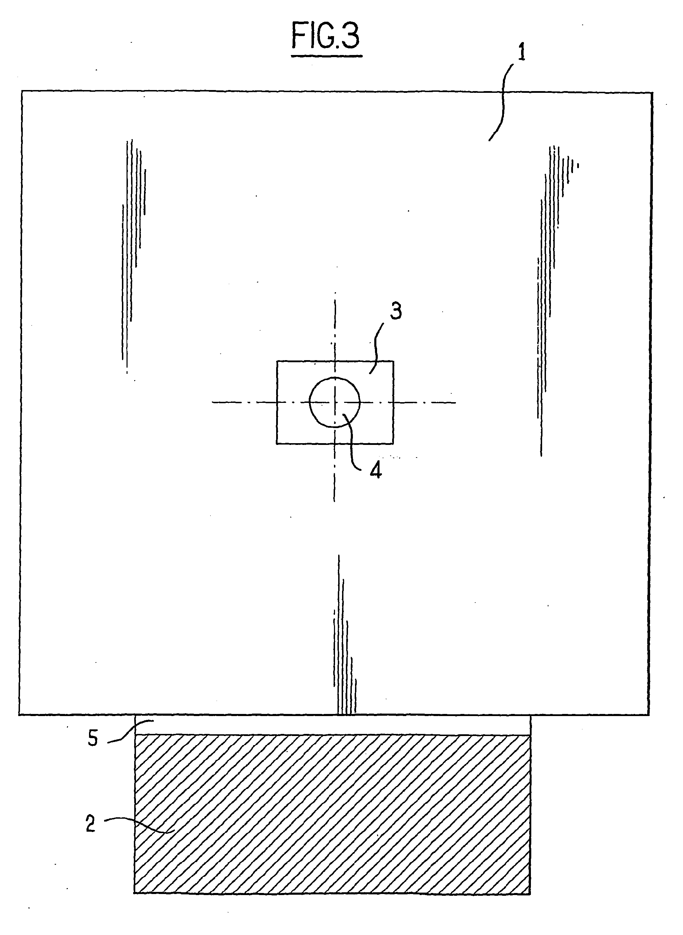

La figure 3 montre une variante de réalisation du champ dans lequel le champ principal est d'environ 3 x 3 mètres et le champ supplémentaire d'environ de 2 x 1 mètres.

De fait, l'invention n'est pas limitée à des dimensions particulières et les dimensions indiquées ne sont que des dimensions préférées.

Le principe du champ consiste donc en ce que l'eau saline injectée durant l'arthroscopie tombe le long du champ jusqu'à une bande de champ très absorbante située sous les pieds du chirurgien, et isolée du reste du champ par une barrière antibactérienne (bande en polyester, par exemple).

On remarque que les gels super absorbants (polyacrylates, sels d'ammonium, quaternaire, ...) une fois gonflés d'eau, peuvent être glissants. Le chirurgien peut tomber.

De préférence, dans une variante de réalisation (figure 4), on constitue le champ supplémentaire en trois couches : un film imperméable (7) côté sol, contrecollé avec non-tissé (8) très absorbant, contrecollé lui-même avec non-tissé voie sèche (9). L'ensemble représente un poids d'environ 185 g/m², est très absorbant et non glissant.

Le film imperméable (7) est par exemple en polyéthylène. Le non-tissé très absorbant (8) et de préférence du type Airlaid.

L'Airlaid est un non-tissé composé de fibres longues en cellulose, enchevêtrées les unes aux autres, très absorbant (et utilisé déjà en angiographie mais avec un grammage par mètre carré plus faible, 80 à 85 gr/m²), légèrement plus rigide.

Le non tissé voie sèche est composé de fibres courtes en polyester ou cellulose liées chimiquement. Il est moins absorbant que l'Airlaid, et évite l'impression de marcher sur une éponge.

L'invention n'est pas limitée aux réalisations qui ont été décrites.

## Revendications

1. Dispositif pour recueillir l'eau qui est injectée dans une articulation au cours d'une arthroscopie, qui comporte un champ principal (1) d'une surface suffisante pour recouvrir l'articulation et descendre sensiblement jusqu'au niveau du sol lorsque le patient est couché sur le dos sur une table, et qui présente une zone (3) en matériau élastique qui détermine une fenêtre (4) pour le passage de l'articulation au travers de la fenêtre avec étanchéité latérale, le dispositif comportant également un champ supplémentaire (2) très hydrophile qui prolonge le champ principal vers le bas pour récupérer au sol l'eau saline qui a été injectée dans l'articulation, **caractérisé en ce** le champs supplémentaire (2) est séparé du champ principal par une barrière (5) pour les germes.

2. Dispositif selon la revendication 1 dont le champ supplémentaire a une capacité d'absorption d'eau d'environ 3 à 4 litres d'eau par m² de champ.

3. Dispositif selon la revendication 2 et dont le champ supplémentaire (2) présente sur trois côtés des rabats (6) formant gouttières.

4. Dispositif selon la revendication 3 dont les rabats (6) sont fixés au champ supplémentaire (2) par des points de soudure ou de colle.

5. Dispositif selon l'une des revendications 1 à 4 dont le champ supplémentaire (2) est isolé du champ principal (1) par une bande (5) de feuille en polyéthylène.

6. Dispositif selon l'une des revendications 1 à 5 dont le champ supplémentaire (2) comporte un film hydrophobe et une couche très hydrophile rapportée et fixée sur le film.

7. Dispositif selon la revendication 6 dont la couche très hydrophile est un réseau de fibres enchevêtrées associées à un milieu superabsorbant en poudre.

8. Dispositif selon la revendication 7 dont le milieu superabsorbant est un polyacrylate ou un sel d'ammonium quaternaire.

9. Dispositif selon l'une des revendications 1 à 8 dont le champ supplémentaire (2) est constitué par un film imperméable (7), contrecollé avec non-tissé très absorbant (8) lui-même contrecollé avec un non tissé voie sèche (9).

10. Dispositif selon la revendication 9 dont le film imperméable (7) du champ supplémentaire (2) est en polyéthylène.

11. Dispositif selon l'une des revendications 9 et 10 dont le non-tissé voie sèche (9) du champ supplémentaire est composé de fibres longues en cellulose, enchevêtrées les unes aux autres.

12. Dispositif selon l'une des revendications 1 à 11 dont le champ principal (1) est constitué d'un film imperméable sur lequel est collée une feuille d'un matériau non tissé légèrement absorbant.

13. Dispositif selon l'une des revendications 1 à 12 dont le champ principal (1) a une surface d'environ 2 x 3 mètres ou d'environ 3 x 9 mètres.

14. Dispositif selon l'une des revendications 1 à 13 dont le champ supplémentaire (2) a une surface d'environ 0,40 x 2 mètres ou d'environ 0, 40 x 1 mètre.

15. Dispositif selon l'une des revendications 1 à 14 dont la bande (5) intermédiaire entre les deux champs a une largeur d'environ 0, 10 mètre.

16. Dispositif selon l'une des revendications 1 à 15 constitué d'une feuille de base en polyéthylène agencée pour constituer le champ principal, la barrière anti- germes et le champ supplémentaire.

## Claims

1. A device for collecting water which is injected into a joint during an arthroscopy, which includes a main field (1) with sufficient surface area for covering the joint and substantially extending downwards to ground level when the patient is lying on his/her back on a table, and which has an area (3) in elastic material which determines a window (4) for letting the joint through the window with a lateral seal, the device also including an additional very hydrophilic field (2) which extends the main field downwards in order to recover at the ground the saline water which was injected into the joint, **characterized in that** the additional field (2) is separated from the main field by a barrier (5) for the germs.

2. The device according to claim 1, whereof the additional field has a water absorption capacity of about 3 to 4 liters of water per square meter of field.

3. The device according to claim 2, whereof the additional field (2) has on three sides flaps (6) forming gutters.

4. The device according to claim 3, whereof the flaps (6) are attached to the additional field (2) by soldering or adhesive spots.

5. The device according to any of claims 1 to 4, whereof the additional field (2) is isolated from the main field (1) by a polyethylene sheet tape (5).

6. The device according to any of claims 1 to 5, whereof the additional field (2) includes a hydrophobic film and a very hydrophilic layer added and attached onto the film.

7. The device according to claim 6, whereof the very hydrophilic layer is a lattice of entangled fibers associated with a super absorbent powder medium.

8. The device according to claim 7, whereof the super absorbent medium is polyacrylate or a quaternary ammonium salt.

9. The device according to any of claims 1 to 8, whereof the additional field (2) is formed by a watertight film (7), laminated with a very absorbent non-woven (8) itself laminated with a dry air-laid non-woven (9).

10. The device according to claim 9, whereof the watertight film (7) of the additional field (2) is in polyethylene.

11. The device according to any of claims 9 and 10, whereof the dry air-laid non-woven (9) of the additional film consists of long cellulose fibers, entangled with each other.

12. The device according to any of claims 1 to 11, whereof the main field (1) consists of a watertight film on which a sheet of a slightly absorbent non-woven material is adhesively bonded.

13. The device according to any of claims 1 to 12, whereof the main field (1) has a surface area of about 2 x 3 meters or about 3 x 9 meters.

14. The device according to any of claims 1 to 13, whereof the additional field (2) has a surface area of about 0.40 x 2 meters or about 0.40 x 1 meter.

15. The device according to any of claims 1 to 14, whereof the intermediate tape (5) between both fields has a width of about 0.10 meter.

16. The device according to any of claims 1 to 15, consisting of a polyethylene base sheet arranged so as to form the main field, the anti-germ barrier and the additional field.

## Patentansprüche

1. Vorrichtung zum Auffangen des Wassers, das während einer Arthroskopie in ein Gelenk injiziert wird, die ein Hauptabdecktuch (1) umfasst, das eine ausreichende Fläche hat, um das Gelenk abzudecken und im Wesentlichen bis auf die Höhe des Bodens herabzureichen, wenn der Patient auf dem Rücken auf einem Tisch liegt, und die eine Zone (3) aus einem elastischen Material aufweist, die ein Fenster (4) zum Durchführen des Gelenks durch das Fenster hindurch mit seitlicher Abdichtung festlegt, wobei die Vorrichtung ebenfalls ein zusätzliches Tuch (2) umfasst, das stark hydrophil ist und das das Hauptabdecktuch nach unten verlängert, um am Boden das salzhaltige Wasser zu sammeln, das in das Gelenk injiziert wurde, **dadurch gekennzeichnet, dass** das zusätzliche Tuch (2) von dem Hauptabdecktuch durch eine Barriere (5) für Keime getrennt ist.

2. Vorrichtung nach Anspruch 1, deren zusätzliches Tuch eine Wasseraufnahmekapazität von etwa 3 bis 4 Liter Wasser pro m² Tuch hat.

3. Vorrichtung nach Anspruch 2, deren zusätzliches Tuch (2) auf drei Seiten Umschlagsäume (6) hat, die Rinnen bilden.

4. Vorrichtung nach Anspruch 3, deren Umschlagsäume (6) am zusätzlichen Tuch (2) durch Klebe- oder Schweißpunkte befestigt sind.

5. Vorrichtung nach einem der Ansprüche 1 bis 4, deren zusätzliches Tuch (2) vom Hauptabdecktuch (1) durch einen Streifen (5) aus Polyethylenfolie isoliert ist.

6. Vorrichtung nach einem der Ansprüche 1 bis 5, deren zusätzliches Tuch (2) folgendes umfasst: einen hydrophoben Film sowie eine stark hydrophile Schicht, die auf dem Film aufgebracht und befestigt ist.

7. Vorrichtung nach Anspruch 6, deren stark hydrophile Schicht ein Geflecht aus ineinander verschlungenen Fasern, verbunden mit einem superabsorbierenden pulverförmigen Stoff ist.

8. Vorrichtung nach Anspruch 7, deren superabsorbierender Stoff ein Polyacrylat oder ein quaternäres Ammoniumsalz ist.

9. Vorrichtung nach einem der Ansprüche 1 bis 8, deren zusätzliches Tuch (2) durch einen undurchlässigen Film (7) gebildet wird, der mit einem stark absorbierenden Vlies (8) beklebt ist, das wiederum mit einem trocken hergestellten Vlies (9) beklebt ist.

10. Vorrichtung nach Anspruch 9, deren undurchlässiger Film (7) des zusätzlichen Tuchs (2) aus Polyethylen ist.

11. Vorrichtung nach einem der Ansprüche 9 und 10, deren trocken hergestelltes Vlies (9) des zusätzlichen Tuchs aus langen Zellulosefasern besteht, die ineinander verschlungen sind.

12. Vorrichtung nach einem der Ansprüche 1 bis 11, deren Hauptabdecktuch (1) aus einem undurchlässigen Film besteht, auf den eine Folie aus einem leicht absorbierenden Vliesstoff geklebt ist.

13. Vorrichtung nach einem der Ansprüche 1 bis 12, deren Hauptabdecktuch (1) eine Fläche von etwa 2 x 3 Meter oder von etwa 3 x 9 Meter hat.

14. Vorrichtung nach einem der Ansprüche 1 bis 13, deren zusätzliches Tuch (2) eine Fläche von etwa 0,40 x 2 Meter oder von etwa 0,40 x 1 Meter hat.

15. Vorrichtung nach einem der Ansprüche 1 bis 14, deren zwischen den beiden Tüchern angeordneter Streifen (5) eine Breite von etwa 0,10 Meter hat.

16. Vorrichtung nach einem der Ansprüche 1 bis 15, die aus einer Basisfolie aus Polyethylen besteht, die derart ausgeführt ist, dass sie das Hauptabdecktuch, die Antikeim-Barriere und das zusätzliche Tuch bildet.
